# EUROPÄISCHE PATENTSCHRIFT

(11) **EP 3 583 421 B1**
(45) Veröffentlichungstag und Bekanntmachung des Hinweises auf die Patenterteilung: **09.02.2022**
(21) Anmeldenummer: 17842354.7
(22) Anmeldetag: 28.11.2017
(51) Int. Cl.: G01N 33/50

(54) **NEUES IN-VITRO VERFAHREN ZUR WIRKSAMKEITSVORHERSAGE VON MEDIKAMENTENKOMBINATIONEN IN ZIELZELLEN; INSBESONDERE TUMORZELLEN**
NOVEL IN VITRO SYSTEM FOR PREDICTING THE EFFICACY OF DRUG COMBINATIONS IN TUMOR CELLS OR OTHER TARGET CELLS
NOUVEAU SYSTÈME IN VITRO PERMETTANT DE PRÉDIRE L'EFFICACITÉ DE COMBINAISONS DE MÉDICAMENTS DANS DES CELLULES TUMORALES OU AUTRES CELLULES CIBLES

(30) Priorität: 04.10.2016 DE 102016011765
(43) Veröffentlichungstag der Anmeldung: 25.12.2019
(73) Patentinhaber: Kischkel, Frank ,Christian, 69120 Heidelberg (DE)
(72) Erfinder: Kischkel, Frank ,Christian, 69120 Heidelberg (DE)
(86) Internationale Anmeldenummer: PCT/EP2017/001383
(87) Internationale Veröffentlichungsnummer: WO 2018/065097

(56) Entgegenhaltungen:
- WO-A1-2010/051552
- WO-A2-2008/095049
- FRANK CHRISTIAN KISCHKEL ET AL: "Conclusion", PEERJ, Bd. 5, 2. März 2017 (2017-03-02), Seite e3030, XP055462459, DOI: 10.7717/peerj.3030
- CARL-OLOF HILLERDAL ET AL: "<i>Ex vivo</i> evaluation of tumor cell specific drug responses in malignant pleural effusions", ONCOTARGET, Bd. 8, Nr. 47, 10. Oktober 2017 (2017-10-10), Seiten 82885-82896, XP055462915, DOI: 10.18632/oncotarget.20889
- Anonym: "Standard Operating ProcedureSOP_All_B735_en_AA04 Clinical Evaluation of CTR-Test®", TherapySelect , 13. Juni 2017 (2017-06-13), XP002779574, Gefunden im Internet: URL:http://www.therapyselect.de/sites/defa ult/files/downloads/ctr-test/ctr-test_clin ical-evaluation_en.pdf [gefunden am 2018-03-26]
- ALEKSANDAR RADUJKOVIC ET AL: "In vitro testing of drug combinations employing nilotinib and alkylating agents with regard to pretransplant conditioning treatment of advanced-phase chronic myeloid leukemia", CANCER CHEMOTHERAPY AND PHARMACOLOGY., Bd. 74, Nr. 2, 20. Juli 2014 (2014-07-20), Seiten 427-432, XP055462921, BERLIN. ISSN: 0344-5704, DOI: 10.1007/s00280-014-2533-6
- M. KASHIF ET AL: "In vitro discovery of promising anti-cancer drug combinations using iterative maximisation of a therapeutic index", SCIENTIFIC REPORTS, Bd. 5, Nr. 1, 22. September 2015 (2015-09-22), XP055462923, DOI: 10.1038/srep14118
- JULIE FOUCQUIER ET AL: "Analysis of drug combinations: current methodological landscape", PHARMACOLOGY RESEARCH & PERSPECTIVES, Bd. 3, Nr. 3, E00149, 1. Juni 2015 (2015-06-01), Seiten 1-11, XP055462926, GB ISSN: 2052-1707, DOI: 10.1002/prp2.149
- T.-C. CHOU: "Drug Combination Studies and Their Synergy Quantification Using the Chou-Talalay Method", CANCER RESEARCH, Bd. 70, Nr. 2, 15. Januar 2010 (2010-01-15), Seiten 440-446, XP055169871, ISSN: 0008-5472, DOI: 10.1158/0008-5472.CAN-09-1947

## Beschreibung

### Beschreibung

Es ist bekannt, dass sich das Ansprechen, bzw. Nichtansprechen von chemotherapeutischen Medikamenten für Krebspatienten mittels individuellen in vitro Testungen an lebenden Tumorzellen vor oder während der Therapie ermitteln lassen kann (in vitro Testungen). Hierzu werden lebende Tumorzellen außerhalb des Patienten mit unterschiedlichen chemotherapeutischen Medikamenten (z.B. Paclitaxel, Topotecan, Doxorubicin, Gemcitabin oder andere) einzeln oder in Kombination inkubiert. Ob und wie stark das jeweilige chemotherapeutische Medikament oder die Medikamentenkombination eine zytotoxische oder zytostatische Wirkung auf die Tumorzellen hat, läßt sich durch unterschiedliche Verfahren auslesen. Beispiele sind der Thymidin-Einbau-Test, "Colony-Forming-Assay", der DiSC-Test, ein Test basierend auf der Detektion der Ansäuerungsfähigkeit von Zellen (Biosensorchip-Test) oder der ATP-Test (Weisenthal et al., 1983; Kern & Weisenthal, 1990; Weisenthal & Kern, 1991; Waldenmaier, Babarina & Kischkel, 2003; Neubauer et al., 2008; Patent WO 2008/095049). Alternativ zu den in vitro Testsystemen gibt es die Möglichkeit die Wirksamkeit der Medikamente durch Biomarker-Testungen zu ermitteln. Biomarker sind alle Informationen, die man aus den Zellen oder Gewebeproben ermitteln kann. Hierzu zählen Proteine oder Proteinaktivitäten, RNA oder DNA Informationen.

In der Praxis finden aber mehr und mehr Kombinationstherapien Anwendung, wobei zwei oder mehr Medikamente zeitgleich oder während eines Therapiezyklus kombiniert werden. Um die Wirksamkeit dieser Kombinationstherapie richtig vorher zu sagen, fehlt es an einem geeigneten Verfahren. Der Stand der Technik ist, dass die Wirksamkeit der einzelnen Medikamente separat in vitro getestet wird. Dabei gilt die Prämisse, dass sich die wirksamste Kombinationstherapie aus den wirksamsten Einzelmedikamenten zusammensetzt. Oder anders ausgedrückt, wenn eines der Medikamente keine oder eine schlechte Wirksamkeit in oben genannten Testverfahren zeigt, so ist es nicht die beste Kombinationswirkungen. Dabei wird die mögliche synergistische Wirkung, die Medikamente haben können, nicht berücksichtigt. Der gegenteilige Effekt von antagonistischen Wirkungen wird dabei ebenso nicht berücksichtigt.

Neben der Messung der Einzelsubstanzen gibt es keine gute Alternative für die Messung von Wirksamkeiten von Kombinationstherapien, die einfach anzuwenden ist und für die klinische Anwendung eine Rolle spielen. In der Literatur findet man Ansätze, wobei ein Medikament in der Konzentration konstant gehalten wird und das andere Medikament dagegen titriert wird. Eine andere Alternative beschäftigt sich mit dem Kombinationsindex (Combinational Index (CI)) oder Therapeutic Index (TI), um synergistische und antagonistische Effekte zu bestimmen (Chou, 2010;Radujkovic et al., 2014; Kasif et al., 2015). Schließlich versucht man auch mit der Berücksichtigung von klinischen Ansprech- und Überlebensdaten eine Vorhersage zu erreichen (Patent WO 2010/051552).

Es ist für die Wirksamkeitsmessung in vitro Stand der Technik, dass man die Wirksamkeit von Einzelsubstanzen über die Messung einer Medikamentenkonzentration bestimmen kann, die die beste Verteilungskurve der Wirksamkeiten über eine größere Anzahl von Patientengeweben zeigt (Patent WO 2008/095049).

Die mit der Erfindung erzielten Vorteile bestehen nun darin, die Bedingungen für die Einzelmessungen zu nutzen und damit Aussagen über die Kombinationswirkungen machen zu können. Damit sollen insbesondere die speziell zugeschnittene Therapie für den einzelnen Patienten für Kombinationstherapien verbessert werden und dadurch die Behandlungserfolge gezielt erhöht werden, wodurch das Gesundheitssystem Kosten einsparen kann.

Eine vorteilhafte Ausgestaltung der Erfindung ist in den Unteransprüchen angegeben.

Dadurch könnte man die hier dargestellte Austestung von Therapiekombinationen auf alle Krebsmedikamente oder Therapien ausweiten, die auf die lebenden Tumorzellen einen Einfluss haben und die lebende Tumorzellen als Auslesefaktor benötigen.

### Ein Ausführungsbeispiel der Erfindung ist im Folgenden beschrieben

Zur Ermittlung der Wirksamkeit von Chemotherapeutika wird das in vitro Testverfahren CTR-Test verwendet, in dem die Wirksamkeit über die Prozent *Cell Growth Inhibition* (PCI) bestimmt wird. Die Parameter für die Ermittlung der Wirksamkeiten werden für die Einzelsubstanzen ermittelt. Zu den Parametern gehören
1. die beste Konzentration der Einzelsubstanzen für deren Wirksamkeitsmessung als Einzelsubstanz und
2. deren Mittelwerte (und Median-Werte) und Standardabweichungen der Verteilungskurve der Wirksamkeit einer Einzelsubstanz. (Dadurch lassen sich die Wirksamkeiten der Substanzen ermitteln.)

Die Wirksamkeit für einzelne Patientenproben kann nun z.B. durch die Einteilung der Verteilungskurve in bestimmte Bereiche ermittelt werden, wie es beim CTR-Test der Fall ist.

Die Wirksamkeitstestung von einer Kombinationsmessung mit zwei Substanzen geschieht durch die Verwendung von beiden Medikamenten, aber mit jeweils der halben ermittelten besten Konzentration. Für die Messung von drei Substanzen werden jeweils 1/3 der Konzentrationen genommen. Für die Messung von vier Substanzen ein ¼ und so weiter. Die Wirksamkeit der Kombinationsmessung wird nun entweder ebenfalls über die Mittelwerte (und Median-Werte) und Standardabweichungen der neuen Verteilungskurve der Wirksamkeit der Kombination ermittelt oder es werden die jeweiligen Mittelwerte (Median-Werte) und jeweiligen Standardabweichungen der Verteilungskurve der Einzelsubstanzen verwendet. Für die letztere Alternative werden die Mittelwerte (Median-Werte) und die Standardabweichungen der gemessenen Medikamente zusammen gezählt und durch die Anzahl der gemessenen Medikamente geteilt. Die Verwendung der letzten Methode hat den Vorteil, dass die Wirksamkeit auch von unbekannten Kombinationen ermittelt werden kann. Des weiteren können synergistische und antagonistische Effekte in den Testergebnissen der gemessenen Kombinationen richtig dargestellt werden unter Verwendung der berechneten Grenzen.

### Material und Methoden

### Sammlung von Tumorgewebeproben

Die Tumorproben wurden als Teil des kommerziell angebotenen CTR-Tests oder als Teil einer klinischen Studie gesammelt. Alle berücksichtigten Proben waren übrig nachdem der kommerzielle Test oder der Test für die klinische Studie durchgeführt wurde. Für alle Proben sind Einverständniserklärungen der Patienten vorhanden, welche weitere wissenschaftliche Untersuchungen erlauben. Ingesamt wurden 273 Ovarialkarzinome, 1 malignes Melanom, 1 kleinzelliges Lungenkarzinom, 1 Mammakarzinom, 4 Kolonkarzinome und 1 nicht-kleinzelliges Lungenkarzinom gesammelt. Direkt nach der operativen Entnahme wurden die Gewebeproben in Medium gelagert und ins Labor geschickt (TherapySelect, Heidelberg, Deutschland), um den CTR-Test durchzuführen. Die Proben trafen innerhalb von 24 Stunden bei TherapySelect ein und wurden noch am selben Tag weiter verarbeitet.

### CTR-Test

Die Gewebeproben wurden weiter verarbeitet und der CTR-Test wurde von TherapySelect entsprechend eines publizierten Protokolls durchgeführt (Kern & Weisenthal, 1990; d'Amato et al., 2009). Kurz zusammengefasst, frisches Tumormaterial wird in Einzelzellen und kleinere Zellaggregate (Spheroide) zerkleinert. Die Lebensfähigkeit der Zellen und der Anteil an Tumorzellen wird bestimmt. Die Zellen werden in eine Kulturschale ausgesät und sofort mit einem spezifischen Chemotherapeutikum oder einer Medikamentenkombination behandelt. Nach 72 Stunden Inkubationszeit wird tritiiertes Thymidin (H³-Thymidin) zu den Zellen hinzu gegeben. Nach weiteren 48 Stunden werden die Zellen auf Glasfiber-Filter geerntet und die Isotop-Aufnahme in die DNA wird mit Hilfe eines Szintillationszählers anaylsiert. Zellen, die ohne Medikamente kultiviert wurden, dienen als Negativ-Kontrolle und Zellen, die mit einer letalen Dosis behandelt wurden dienen als Positiv-Kontrolle. Der chemotherapeutische Effekt wird in Prozent *Cell Growth Inhibition* (PCI) gemessen.

### Für die Analyse verwendete Medikamente

Alle Medikamente, die in dieser Studie verwendet wurden, wurden nach ihrer therapeutischen Relevanz ausgewählt und sie wurden für den CTR-Test validiert vor der Analyse der Tumorproben für diese Studie. Für die Validierung wurden verschiedene Medikamenten-Konzentrationen im CTR-Test mit frisch isolierten Tumorproben getestet, um eine Konzentration zu finden, die eine ausreichende Verteilung der Medikamentenwirkung unter den Tumorproben zeigt. Die tatsächlich verwendeten Medikamenten-Konzentrationen werden in Tabelle 3 gezeigt. Für die Messungen der Medikamenten-Kombinationen (zwei Medikamente) wurde die Hälfte der Konzentrationen der einzelnen Medikamente verwendet um die Zellen gleichzeitig zu behandeln.

### Statistische Auswertung

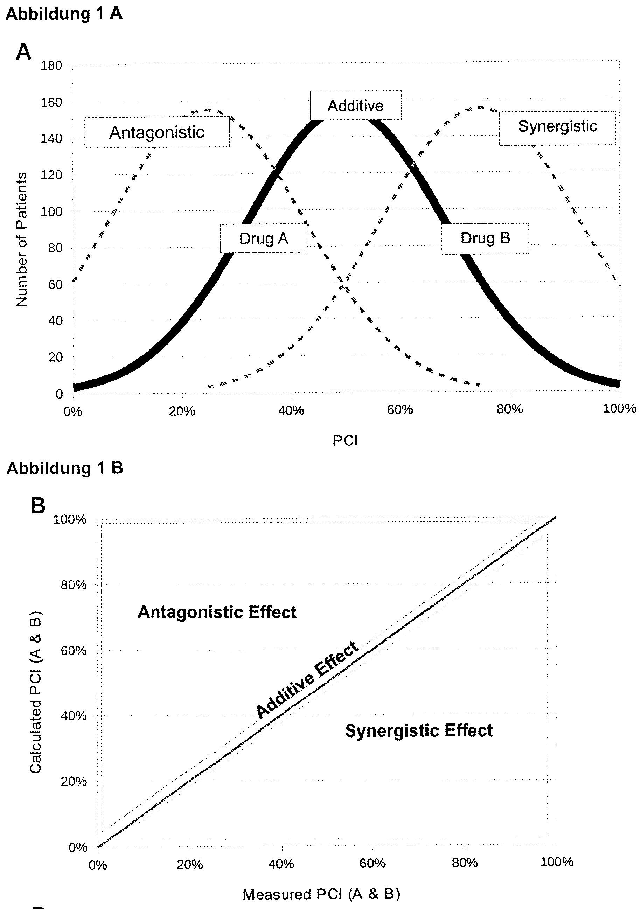

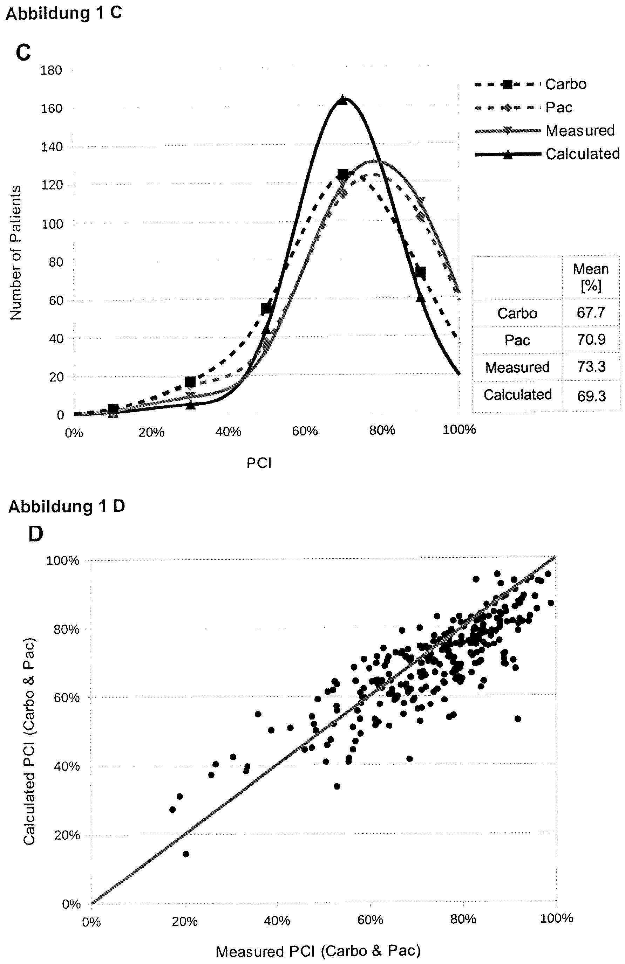

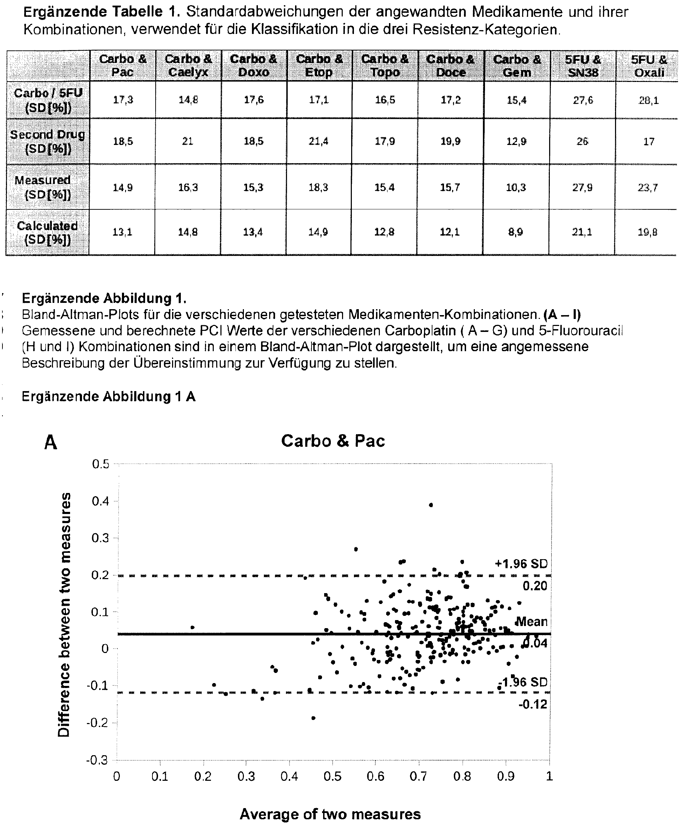

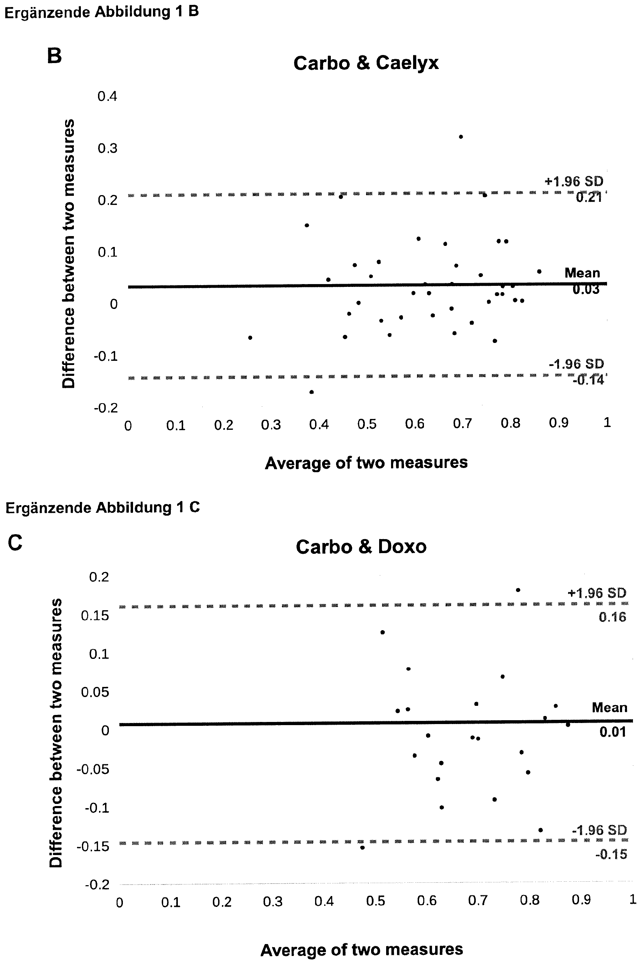

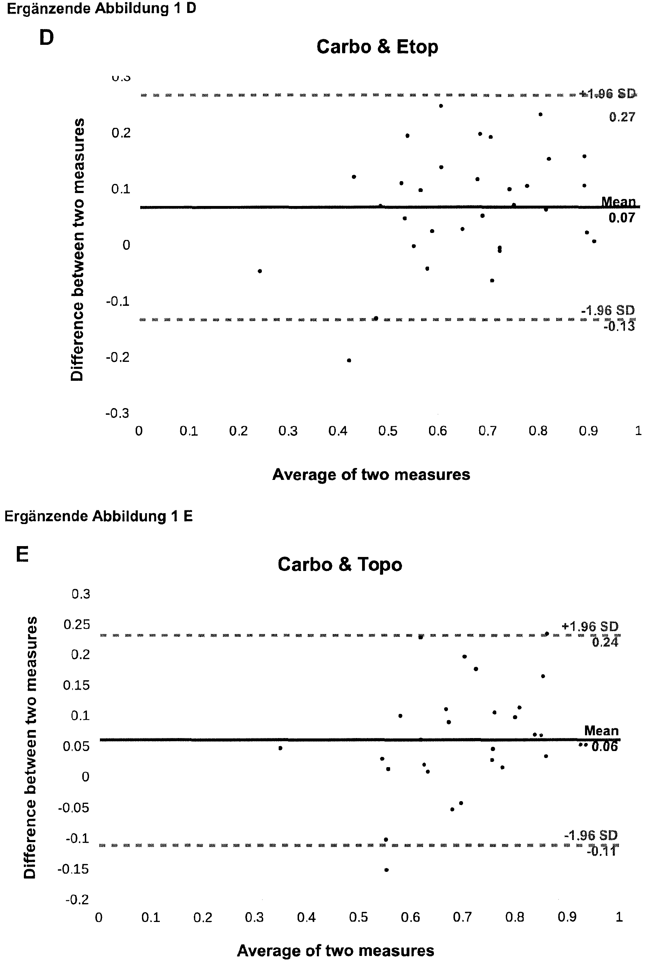

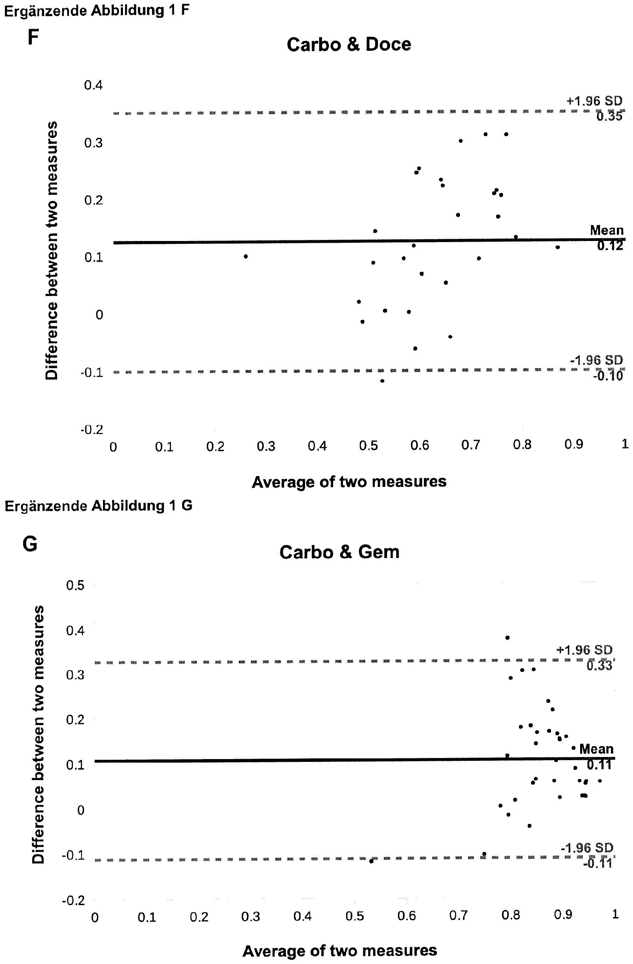

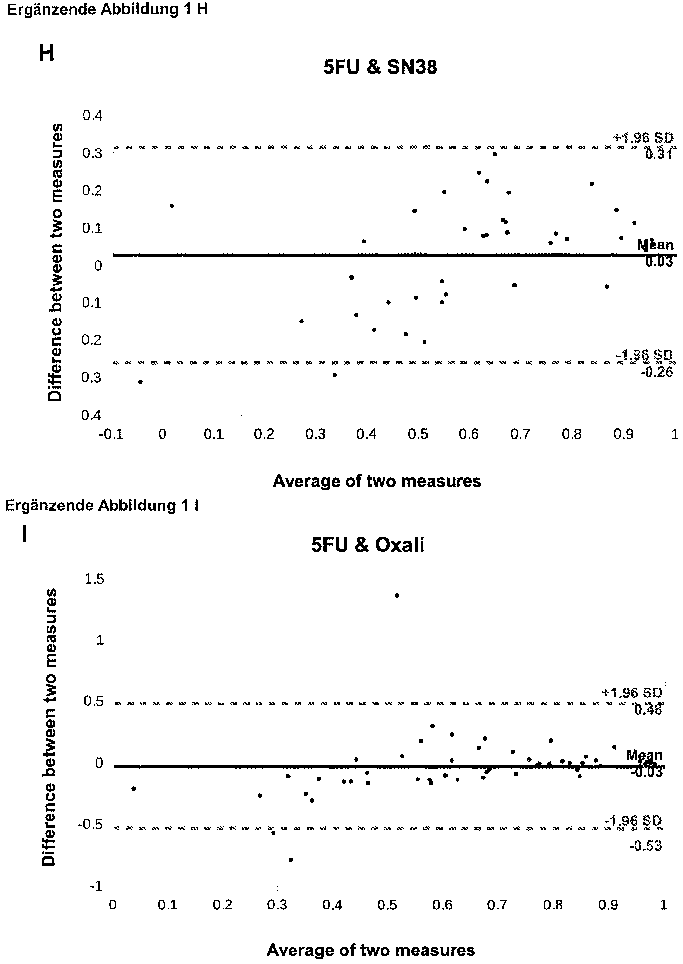

Die PCI Werte wurden anhand der Ansprechrate eines Tumorproben-Kollektivs auf eine bestimmte Konzentration eines Chemotherapeutikums ermittelt. Die Häufigkeitsverteilungen der PCI Werte wurden generiert indem die Mittelpunkte von 4- oder 5- Klassen Histogrammen in Excel durch eine glatte Kurve verbunden wurden. Die Häufigkeitsverteilungen wurden verwendet um die drei Resistenz-Kategorien SR, MR und ER zu identifizieren. Dadurch wurde der Mittelwert (µ) und die Standardabweichung (SD) bestimmt. Die Mittelwerte werden in den entsprechenden Abbildungen gezeigt, die SD Werte findet man in der Ergänzenden Tabelle 1. ER ist charakterisiert als PCI < µ - 1SD, MR als PCI > ER aber < µ und SR ist charakterisiert als PCI ≥ µ. Wir haben die gemessenen und berechneten PCI Werte der unterschiedlichen Kombinationen miteinander verglichen indem der Pearson Korrelationskoeffizient bestimmt wurde. Um die Übereinstimmung zwischen berechneten und gemessenen Werten einzuschätzen, zeigten wir den Unterschied von berechneten und gemessen Werten im Vergleich zum Durchschnitt von beiden Werten in einem Bland-Altman Plot (Ergänzende Abbildung 1).

### Literatur

d'Amato TA., Pettiford BL., Schuchert MJ., Parker R., Ricketts WA., Luketich JD., Landreneau RJ. 2009. Survival among patients with platinum resistant, locally advanced non-small cell lung cancer treated with platinum-based systemic therapy. Annals of Surgical Oncology 16:2848-2855. DOI: 10.1245/s10434-009-0608-0.
Chou T-C. 2010. Drug combination studies and their synergy quantification using the Chou-Talalay method. Cancer Research 70:440-446. DOI: 10.1158/0008-5472.CAN-09-1947.
Kashif M., Andersson C., Hassan S., Karlsson H., Senkowski W., Fryknäs M., Nygren P., Larsson R., Gustafsson M.G. 2015. In vitro discovery of promising anti-cancer drug combinations using iterative maximisation of a therapeutic index. Scientific Reports 5: 14118. DOI: 10.1038/srep14118.
Kern DH., Weisenthal LM. 1990. Highly specific prediction of antineoplastic drug resistance with an in vitro assay using suprapharmacologic drug exposures. Journal of the National Cancer Institute 82:582-588.
Neubauer H., Stefanova M., Solomayer E., Meisner C., Zwirner M., Wallwiener D., Fehm T. 2008. Predicting resistance to platinum-containing chemotherapy with the ATP tumor chemosensitivity assay in primary ovarian cancer. Anticancer Research 28:949-955.
Patent WO 2008/095049. Reagents and Methods for Predicting Drug Resistance
Patent WO 2010/051552. Methods of Simulating Chemotherapy for a Patient
Radujkovic A., Luft T., Dreger P., Ho AD., Zeller WJ., Fruehauf S., Topaly J. 2014. In vitro testing of drug combinations employing nilotinib and alkylating agents with regard to pretransplant conditioning treatment of advanced-phase chronic myeloid leukemia. Cancer Chemother Pharmacol 74(2):427-32. DOI: 10.1007/s00280-014-2533-6.
Waldenmaier DS., Babarina A., Kischkel FC. 2003. Rapid in vitro chemosensitivity analysis of human colon tumor cell lines. Toxicology and Applied Pharmacology 192:237-245.
Weisenthal LM., Kern DH. 1991. Prediction of drug resistance in cancer chemotherapy: the Kern and DiSC assays. Oncology (Williston Park, N.Y.) 5:93-103, 111-114, 117-118.
Weisenthal LM., Marsden JA., Dill PL., Macaluso CK. 1983. A novel dye exclusion method for testing in vitro chemosensitivity of human tumors. Cancer Research 43:749-757.

### Legenden

**Abbildung 1****.** Neues System um Medikamenten-Kombinationen *in vitro* mit dem CTR-Test zu testen. (A) Ideale Verteilung der Prozent *Cell Growth Inhibition* (PCI) Werte aus einem Tumorpatienten-Kollektiv unter Verwendung der idealen Konzentration eines Medikaments (schwarze Kurve, Mittelwert bei 50 % PCI). Zwei einzeln gemessene Medikamente A und B mit idealen Konzentrationen zeigen identische ideale Verteilungskurven (schwarze Kurve). Die eine Hälfte der idealen Konzentration von Medikament A kann durch eine Hälfte der idealen Konzentration von Medikament B ersetzt werden, oder umgekehrt, wenn die Medikamente in Kombination verabreicht werden. Dies resultiert in einer Kurve die identisch ist zu den einzelnen Kurven von A oder B (schwarze Kurve, additiver Effekt). Der Grund für eine Verschiebung der Kurve nach links wäre ein antagonistischer Effekt, für eine Verschiebung nach rechts wäre der Grund ein synergistischer Effekt, wenn zwei Medikamente in Kombination verabreicht werden (graue gestrichelte Kurven). (B) In einer idealen Situation sind die gemessenen PCI Werte einer Medikamenten-Kombination identisch zu den berechneten PCI Werten (Formel: siehe Text). Wenn die idealen PCI Werte gegeneinander geplottet werden, resultiert dies in einem Korrelationskoeffizient von 1 (additiver Effekt). Datenpunkte oberhalb oder unterhalb der Linie zeigen einen antagonistischen beziehungsweise synergistischen Effekt. (C) 273 Ovarialkarzinom-Proben (99 Primärerkrankungen, 140 Rezidive und 34 unbekannte Ovarialkarzinomfälle) wurden mit Carboplatin und Paclitaxel einzeln oder in Kombination behandelt. Die PCI Werte der einzelnen Medikamente wurden mit dem CTR-Test bestimmt. Die PCI Werte der Kombination wurden ebenfalls mit dem CTR-Test gemessen und zusätzlich mit Hilfe der beschriebenen Formel berechnet. Um die Kombination zu messen wurde die Hälfte der Konzentration jedes Medikaments verwendet. Die Häufigkeitsverteilungen der PCI Werte der verschiedenen Situationen wurden geplottet (schwarze gestrichelte Kurve: Carboplatin alleine, graue gestrichelte Kurve: Paclitaxel alleine, graue Kurve: gemessene PCI der Kombination, schwarze Kurve berechnete PCI der Kombination). Der mittlere PCI Wert aller Kurven verteilt sich um 70 %. (D) Die gemessenen und berechneten PCI Werte der Kombination wurden gegeneinander geplottet. Dies resultiert in einem Korrelationskoeffizient von 0,84 (Tabelle 1).

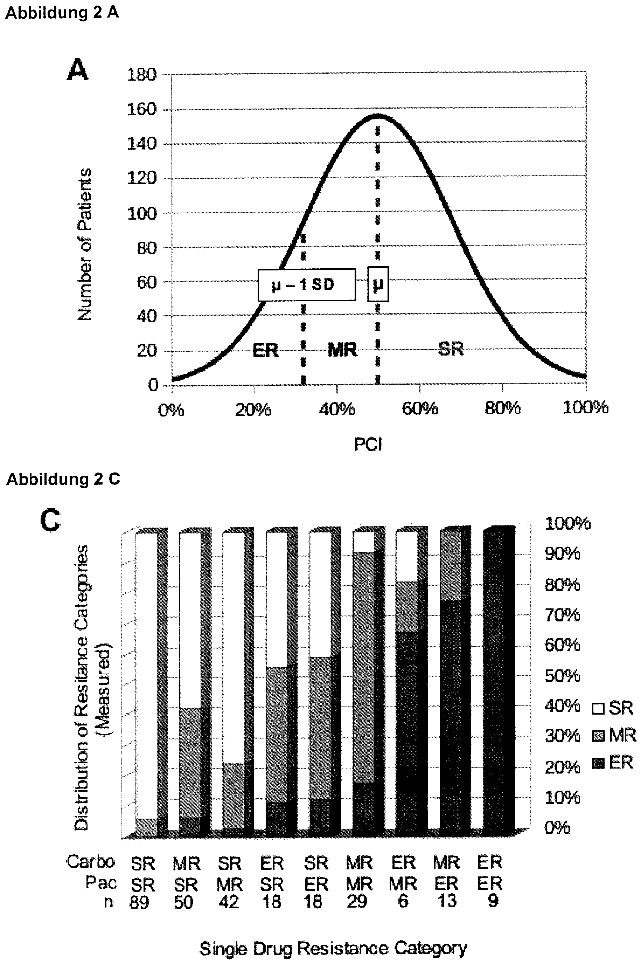

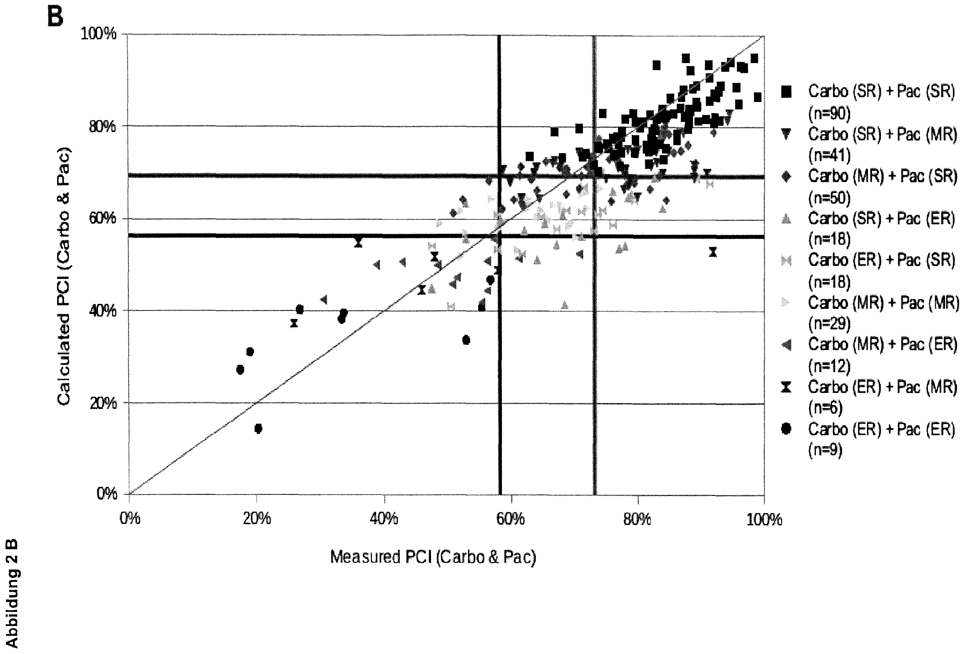

**Abbildung 2****.** Bewertung der Vorhersagekraft des neuen Systems für die Chemoresistenz-Klassifikation. (A) Die Verteilung der PCI Werte eines Tumorpatienten-Kollektivs für ein bestimmtes Medikament verabreicht in einer spezifischen Konzentration wird verwendet um drei Chemoresistenz-Kategorien zu klassifizieren. ER (extreme Resistenz) ist charakterisiert als PCI < µ (Mittelwert) - 1 SD (Standardabweichung) (gestrichelte rote Linie). PCI > ER aber < µ (gestrichelte grüne Linie) ist klassifiziert als MR (mittlere Resistenz), PCI ≥ µ ist klassifiziert als SR (schwache Resistenz). (B und C) Der Datensatz enthält 273 Ovarialkarzinom-Proben. (B) Die Resistenz-Kategorien dieses Datensatzes für Carboplatin alleine, Paclitaxel alleine, gemessene und berechnete Kombination, werden durch das System beschrieben in (A) definiert und die Klassifizierungs-Grenzen für gemessen und berechnet sind markiert durch eine grüne (µ) und rote (µ - 1 SD) Linie. Die zu Grunde liegenden Kurven sind in Abbildung 1 C dargestellt. Diese gemessenen und berechneten PCI Werte der Kombination Carboplatin und Paclitaxel wurden gegeneinander geplottet. Einzelne Datenpunkte sind farblich markiert abhängig von der Chemoresistenz-Kategorie des Patienten für die zwei einzelnen Medikamente. (C) Die Resistenz-Kategorien der einzelnen Medikamente werden mit den gemessenen Kategorien der Kombination Carboplatin und Paclitaxel verglichen.

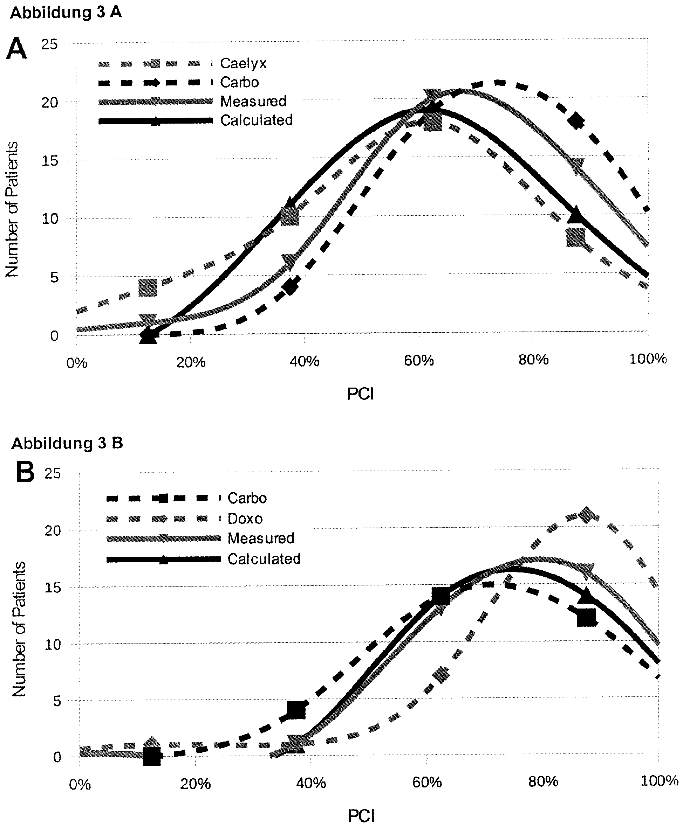

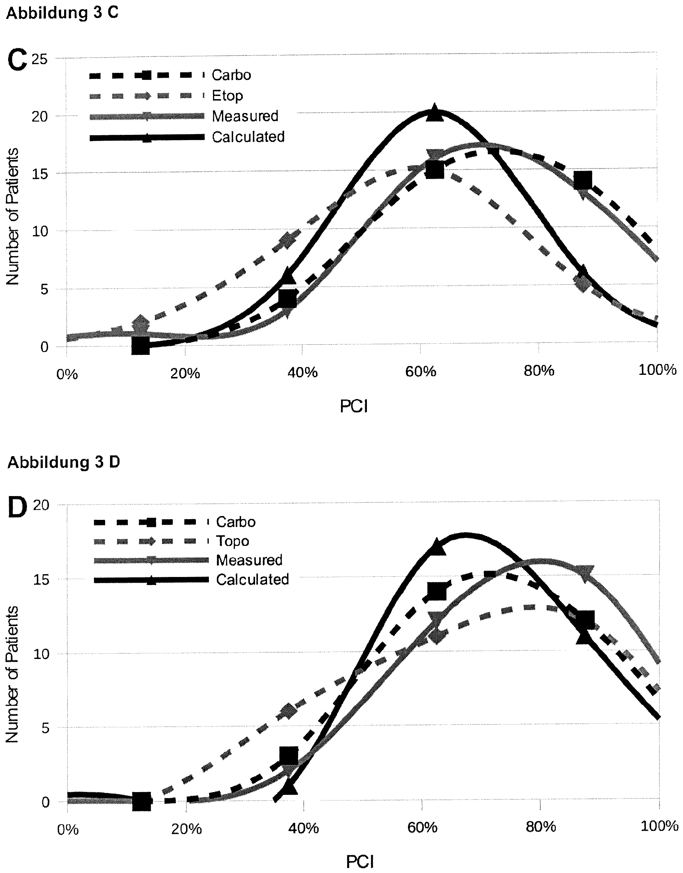

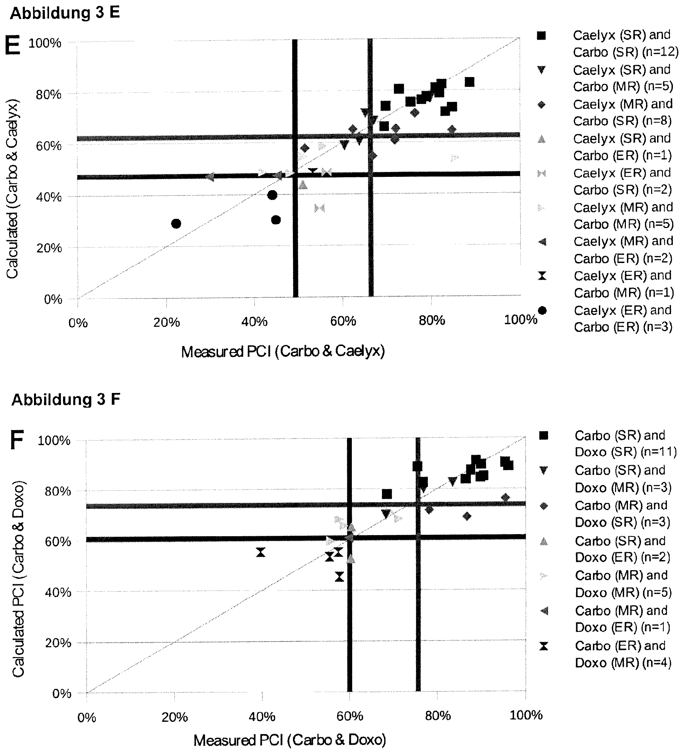

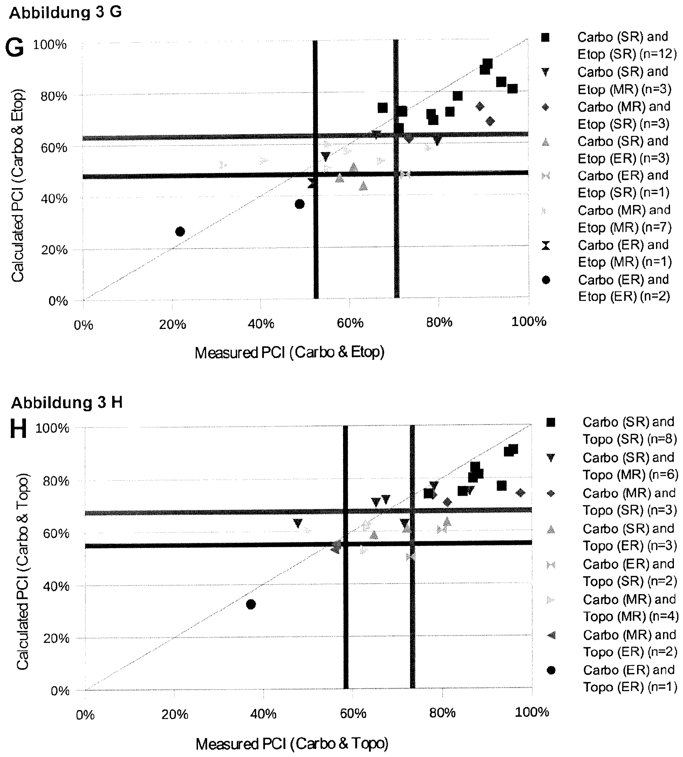

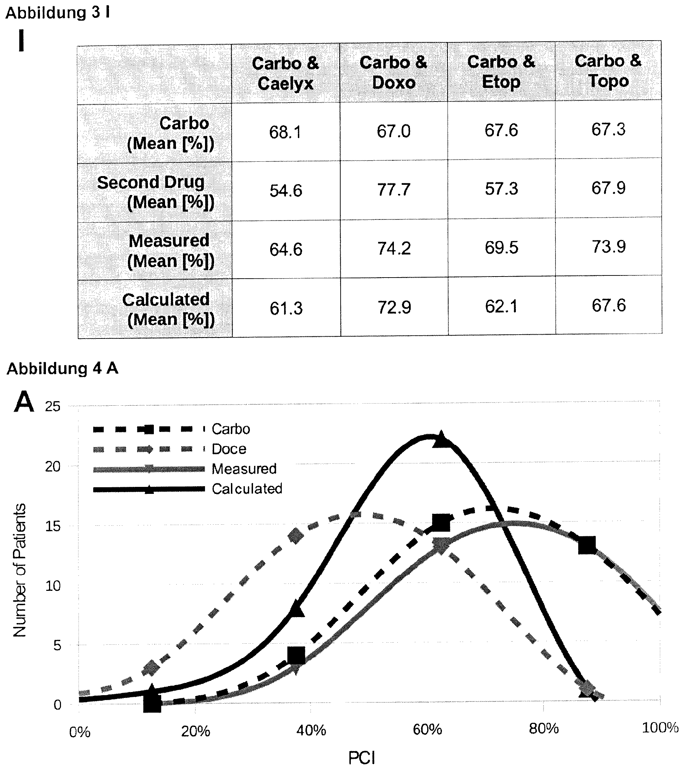

**Abbildung 3****.** Das neue System wird verwendet um andere Medikamenten-Kombinationen zu testen. (A - D) 39 Ovarialkarzinom-Proben wurden mit Carboplatin und Caelyx (A), 29 mit Carboplatin und Doxorubicin (B), 32 mit Carboplatin un Etoposid (C) und 29 mit Carboplatin und Topotecan (D) alleine oder in Kombination behandelt. Der PCI Wert der einzelnen Medikamente und der verschiedenen Kombinationen wurde mit dem CTR-Test gemessen. Zusätzlich wurde der PCI Wert der Kombinationen mit der beschriebenen Formel berechnet. Die Häufigkeitsverteilungen der PCI Werte der verschiedenen Situationen wurden geplottet (schwarze gestrichelte Kurven: Carboplatin alleine, graue gestrichelte Kurven: verschiedene Medikamente alleine, graue Kurven: gemessene PCI der Kombinationen, schwarze Kurven: berechnete PCI der Kombinationen). Die mittleren PCI Werte für alle Kurven sind in I dargestellt. (E - H) Diese Verteilungskurven der PCI Werte für die verschiedenen Medikamente alleine, gemessene und berechnete Kombinationen mit Carboplatin wurden verwendet um Resistenz-Kategorien mit Hilfe des in Abbildung 2A beschriebenen Systems zu definieren. Die Klassifizierungs-Grenzen für gemessen und berechnet sind markiert durch eine grüne (µ) und rote (µ - 1 SD) Linie. Die gemessenen und berechneten PCI Werte der Kombinationen Carboplatin mit einer der vier anderen Medikamente wurden gegeneinander geplottet. Einzelne Datenpunkte sind farblich markiert abhängig von der Chemoresistenz-Kategorie des Patienten für die zwei einzelnen Medikamente. (I) Diese Tabelle zeigt die mittleren PCI Werte für alle Kurven.

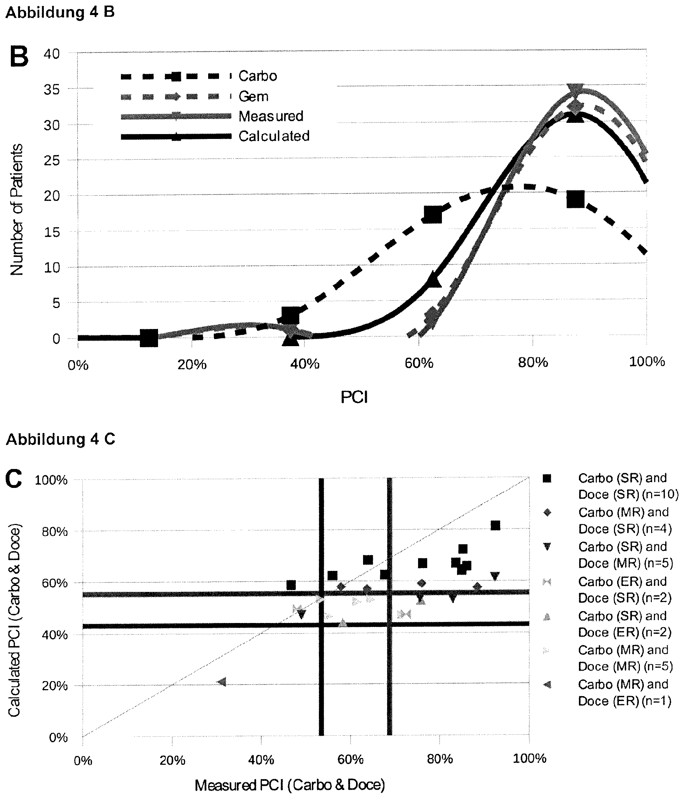

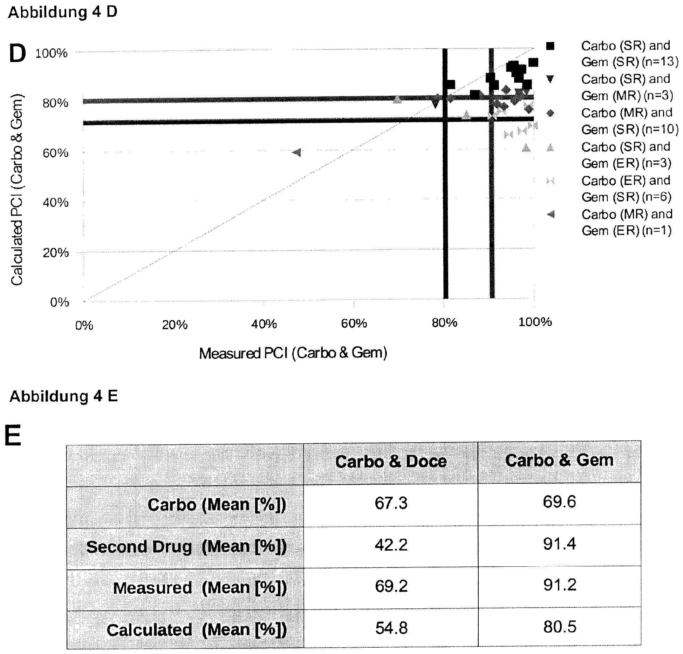

**Abbildung 4****.** Das neue System wird verwendet um andere Medikamenten-Kombinationen zu testen (Ausnahmen). (A und B) 30 Ovarialkarzinom-Proben wurden mit Carboplatin und Docetaxel (A), 36 mit Carboplatin und Gemcitabin (B) alleine oder in Kombination behandelt. Der PCI Wert der einzelnen Medikamente und der beiden Kombinationen wurde mit dem CTR-Test gemessen. Zusätzlich wurde der PCI Wert der Kombinationen mit der beschriebenen Formel berechnet. Die Häufigkeitsverteilungen der PCI Werte der verschiedenen Situationen wurden geplottet (schwarze gestrichelte Kurven: Carboplatin alleine, graue gestrichelte Kurven: Docetaxel oder Gemcitabin alleine, graue Kurven: gemessene PCI der Kombinationen, schwarze Kurven: berechnete PCI der Kombinationen). Die mittleren PCI Werte für alle Kurven sind in E dargestellt. (C und D) Die Verteilungskurven der PCI Werte für die zwei Medikamente alleine, gemessene und berechnete Kombinationen mit Carboplatin wurden verwendet um Resistenz-Kategorien mit Hilfe des in Abbildung 2A beschriebenen Systems zu definieren. Die Klassifizierungs-Grenzen für gemessen und berechnet sind markiert durch eine grüne (µ) und rote (µ - 1 SD) Linie. Die gemessenen und berechneten PCI Werte der Kombinationen Carboplatin mit einer der zwei anderen Medikamente wurden gegeneinander geplottet. Einzelne Datenpunkte sind farblich markiert abhängig von der Chemoresistenz-Kategorie des Patienten für die einzelnen Medikamente. (E) Diese Tabelle zeigt die mittleren PCI Werte für alle Kurven.

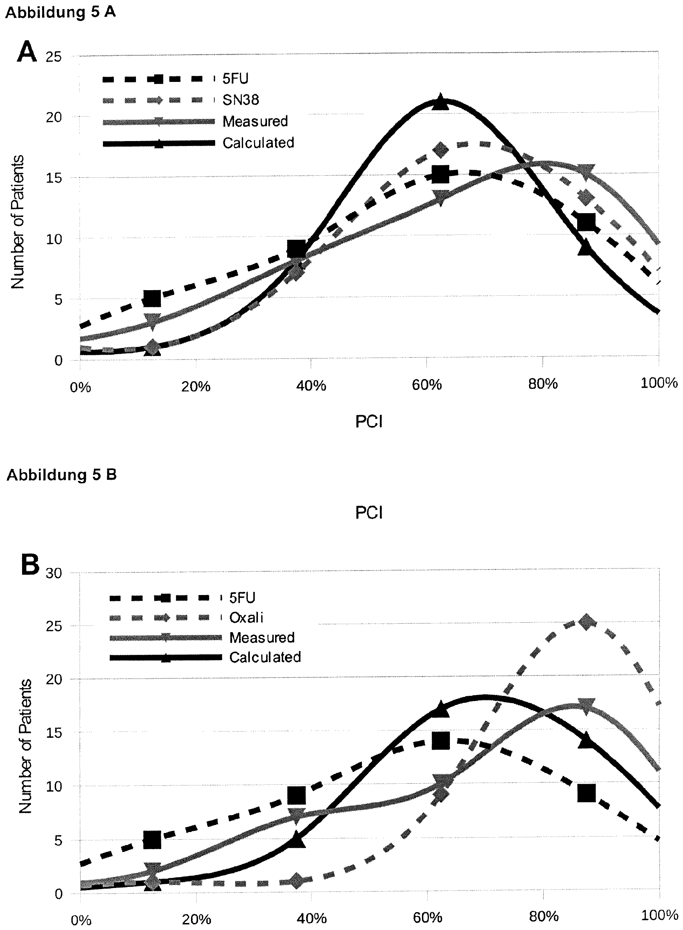

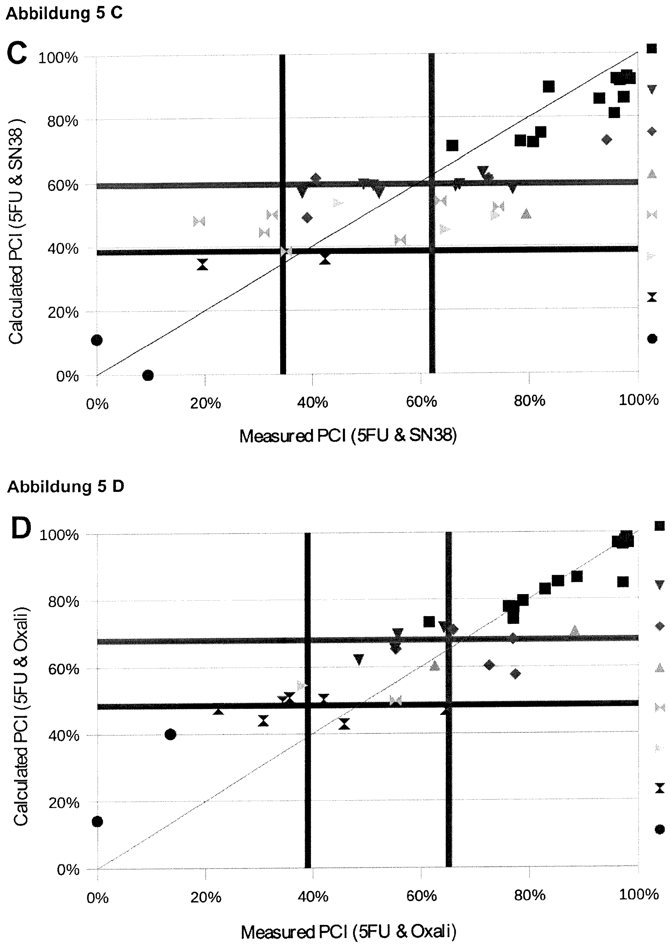

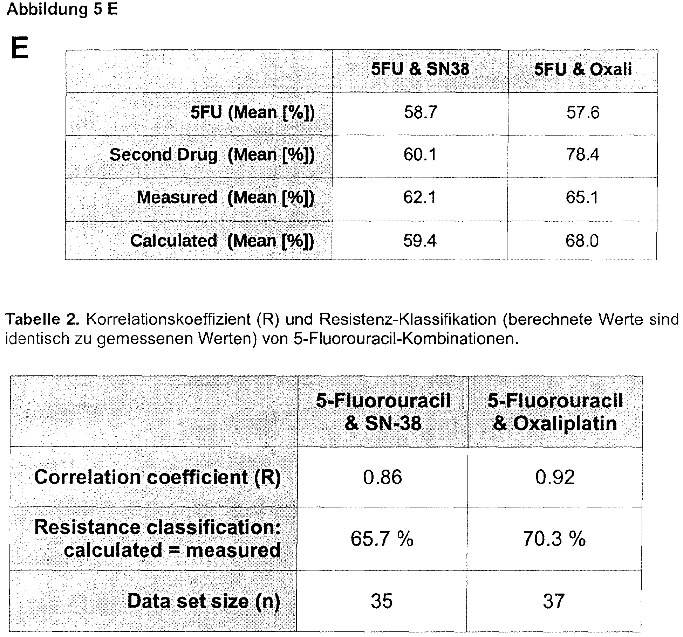

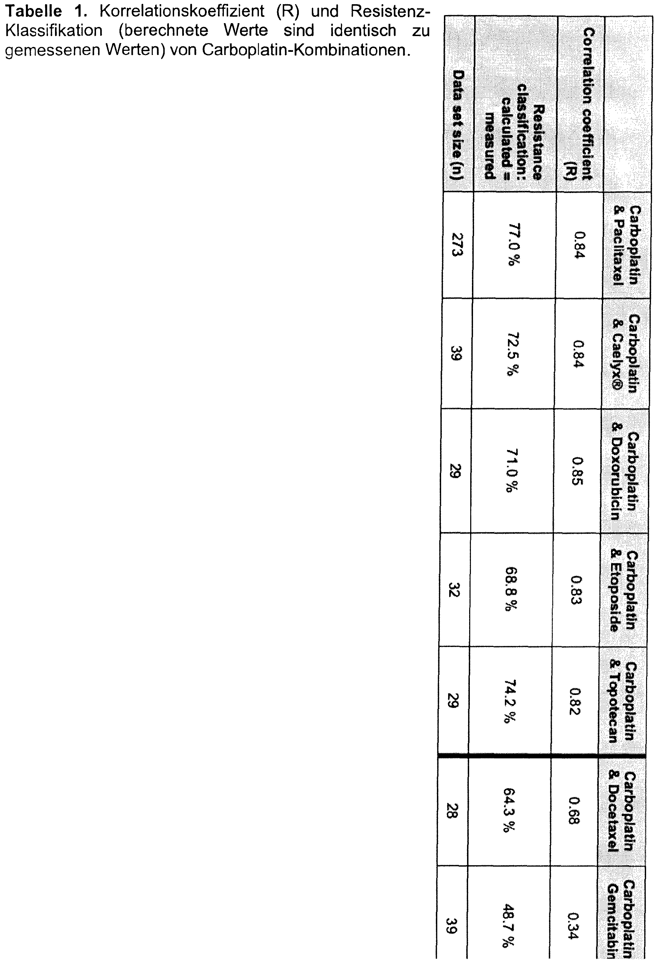

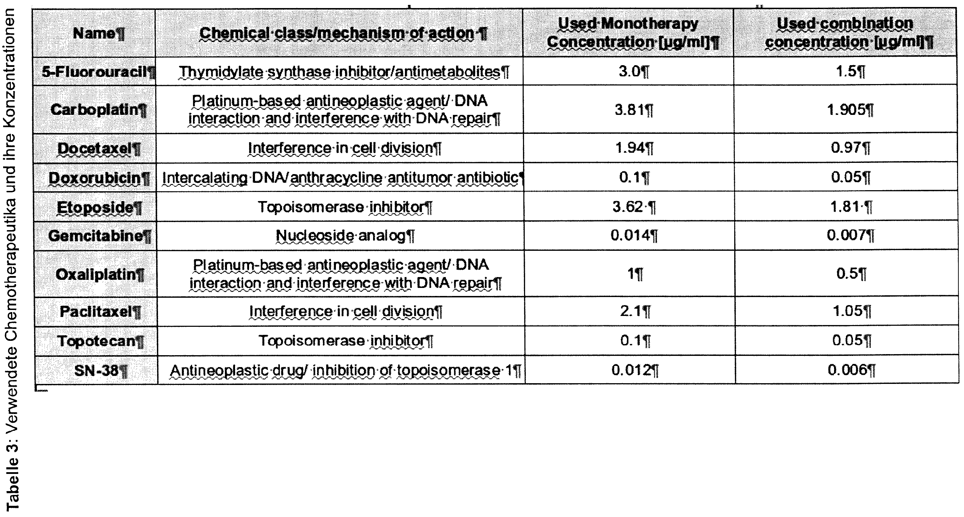

**Abbildung 5****.** Das neue System wird verwendet um andere Carboplatin-unabhängige Medikamenten-Kombinationen zu testen. (A und B) 32 Ovarialkarzinome, 1 Melanom, 1 kleinzelliges Lungenkarzinom, 1 nicht-kleinzelliges Lungenkarzinom, 1 Mammakarzinom und 4 Kolonkarzinome wurden mit 5-Fluorouracil und SN-38 (A) alleine oder in Kombination behandelt; 31 Ovarialkarzinome, 1 Melanom, 1 kleinzelliges Lungenkarzinom, 1 nicht-kleinzelliges Lungenkarzinom, 1 Mammakarzinom und 2 Kolonkarzinome wurden mit 5-Fluorouracil und Oxaliplatin (B) alleine oder in Kombination behandelt. Der PCI Wert der einzelnen Medikamente und der beiden Kombinationen wurde mit dem CTR-Test gemessen und der PCI Wert der beiden Kombinationen wurde mit der beschriebenen Formel berechnet. Die Häufigkeitsverteilungen der PCI Werte der verschiedenen Situationen wurden geplottet (schwarze gestrichelte Kurven: 5-Fluorouracil alleine, graue gestrichelte Kurven: SN-38 oder Oxaliplatin alleine, graue Kurven: gemessene PCI der Kombinationen, schwarze Kurven: berechnete PCI der Kombinationen). Die mittleren PCI Werte für alle Kurven sind in E dargestellt. (C und D) Die Verteilungskurven der PCI Werte für die zwei Medikamente alleine, gemessene und berechnete Kombinationen mit 5-Fluorouracil wurden verwendet um Resistenz-Kategorien mit Hilfe des in Abbildung 2A beschriebenen Systems zu definieren. Die Klassifizierungs-Grenzen für gemessen und berechnet sind markiert durch eine grüne (µ) und rote (µ - 1 SD) Linie. Die gemessenen und berechneten PCI Werte der Kombinationen 5-Fluorouracil mit einer der zwei anderen Medikamente wurden gegeneinander geplottet. Einzelne Datenpunkte sind farblich markiert abhängig von der Chemoresistenz-Kategorie des Patienten für die einzelnen Medikamente. (E) Diese Tabelle zeigt die mittleren PCI Werte für alle Kurven.

## Patentansprüche

1. Verfahren zur Ermittlung und Auswahl der wirksamen oder nichtwirksamen therapeutischen Medikamentenkombinationen für Patienten mittels individuellen in vitro Testungen an lebenden Patientenproben vor oder während der Therapie,
**dadurch gekennzeichnet,**
**dass** die Wirksamkeitsmessung an lebenden Zellen durchgeführt wird,
wobei lebende Zellen entweder aus frischen Tumorgewebeproben oder Gewebeproben anderer Krankheiten oder gesunden Geweben oder Blutproben oder aus Gewebe oder Blut gewonnen und außerhalb des Körpers kultivierten Zellen bestehen und dabei möglichst die ursprünglichen Eigenschaften der Zellen aus dem Körper weiterhin besitzen. Die Zellen können aber auch entsprechend der Behandlung des Patienten außerhalb des Körpers vergleichbar behandelt sein.
**dadurch gekennzeichnet,**
**dass** für die Wirksamkeitsmessung die Einzelmedikamente in deren möglichst optimaler Konzentration vermessen werden und deren Kombinationswirksamkeit rechnerisch kombiniert wird,
wobei die Aussage über die Unwirksamkeit oder Wirksamkeit mit Hilfe der theoretischen Verteilungskurve ermittelt wird
und die optimale Konzentration eines einzelnen Medikaments **dadurch gekennzeichnet ist, dass** die PCIs (Percent Cell Growth Inhibition) oder Daten anderer Wirksamkeitsausleseverfahren unterschiedlich getesteter lebender Zellen einen möglichst breiten PCI-Bereich oder Datenbereich anderer Wirksamkeitsausleseverfahren ergeben
und die theoretische Verteilungskurve sich aus der rechnerischen Kombination der Verteilungskurven aller Einzelsubstanzen, die in der Kombination beteiligt sind, ergibt.
**dadurch gekennzeichnet,**
**dass** die rechnerische Kombination der Wirksamkeiten der Einzelsubstanzen ermittelt wird, in dem deren PCIs (Percent Cell Growth Inhibition) oder Daten andere Wirksamkeitsausleseverfahren summiert und durch die Anzahl der Substanzen der Kombination geteilt wird,
wobei die theoretische Verteilungskurve durch die Berechnung der Mittelwerte für die Mittelwerte, Mediane und Standardabweichungen der Verteilungskurven für die Einzelsubstanzen charakterisiert wird.

2. Verfahren nach Anspruch 1
**dadurch gekennzeichnet,**
**dass** die Wirksamkeit in mindestens zwei Gruppen unterteilt wird,
wobei dies zum Beispiel Wirksamkeit und Unwirksamkeit ist, oder
wobei eine Möglichkeit die Unterteilung in Extreme Resistenz (ER), Mittlere Resistenz (MR) und Schwache Resistenz (SR) ist,
**dadurch gekennzeichnet, dass**
ER: PCIs kleiner Mittelwert (oder Median) - 1 × Standardabweichung sind,
MR: PCIs größer ER aber kleiner Mittelwert (oder Median) sind und
SR: PCIs größer gleich Mittelwert (oder Median)sind
oder
sich die Wirksamkeitsgrenzen und Gruppen auch anderes definieren lassen, aber die Grenzen und Gruppen dann für alle getesteten Medikamente oder Kombinationen gleich angewendet werden.

3. Verfahren nach Anspruch 1 bis 2
**dadurch gekennzeichnet,**
**dass** die in vitro Testung der CTR-Test oder der identische EDR Assay ist.

4. Verfahren nach Anspruch 1 bis 2 oder 1 bis 3
**dadurch gekennzeichnet,**
**dass** die Kombinationsmessung nicht mit Einzelsubstanzen, sondern durch die direkte Messung der kombinierten Substanzen durchgeführt wird,
wobei die kombinierten Substanzen im gleichen Verhältnis und mit reduzierten Konzentrationen zur Testung geben werden,
wobei
bei 2 Medikamenten-Kombinationen jeweils die ½ der Konzentrationen der Einzelsubstanzen verwendet werden,
bei 3 Medikamenten-Kombinationen jeweils 1/3 der Konzentrationen der Einzelsubstanzen,
für 4 Kombinationen ¼, für 5er 1/5 der Konzentrationen und so weiter verwendet werden.

5. Verfahren nach Anspruch 4
**dadurch gekennzeichnet,**
**dass** für die Wirksamkeitsmessung das Verfahren nach Anspruch 64verwendet wird und die Verteilungskurve für die Wirksamkeit mit lebenden Zellen aufgenommen wird, wodurch so für diese Verteilungskurve die Wirksamkeitsgrenzen definiert werden können.

6. Verfahren nach Anspruch 4 bis 5
**dadurch gekennzeichnet,**
**dass** die Wirksamkeit in mindestens zwei Gruppen unterteilt wird,
wobei eine Möglichkeit die Unterteilung in Extreme Resistenz (ER), Mittlere Resistenz (MR) und Schwache Resistenz (SR) ist,
**dadurch gekennzeichnet, dass**
ER: PCIs kleiner Mittelwert (oder Median) - 1 × Standardabweichung sind,
MR: PCIs größer ER aber kleiner Mittelwert (oder Median) sind und
SR: PCIs größer gleich Mittelwert (oder Median) sind
oder
sich die Wirksamkeitsgrenzen und Gruppen auch anderes definieren lassen, aber die Grenzen und Gruppen dann für alle getesteten Medikamente oder Kombinationen gleich angewendet werden.

7. Verfahren nach Anspruch 1 bis 6
zur Ermittlung von synergistischen, additiven und antagonistischen Effekten von Medikamentenkombinationen. Dies wird durch den Vergleich berechnete Wirksamkeit (Anspruch 1 bis 3 und gemessene Wirksamkeit (Anspruch 4 bis 7) ermittelt.

8. Verfahren nach Anspruch 1 bis 7
**dadurch gekennzeichnet,**
**dass** die therapeutische Medikamente zur Gruppe der Chemotherapeutika (Zytostatika) (wie z.B. Carboplatin oder Paclitaxel), zielgerichtete Medikamente (wie z.B. Tyrosinkinaseinhibitoren, humanisierte Antikörper, monoklonale therapeutische Antikörper oder Anti-Hormon-Medikamente wie Tamoxifen) oder Immuntherapeutika (wie z.B. Checkpoint-Medikamente wie Pembrolizumab, Nivolumab oder Atezolizumab) gehören und zur Krebsbehandlung dienen.

## Claims

1. method for determining and selecting effective or non-effective therapeutic drug combinations for patients by means of individual in vitro tests on living patient samples before or during therapy,
**characterized in**
**that** the efficacy measurement is performed on living cells,
wherein living cells consist either of fresh tumor tissue samples or tissue samples from other diseases or healthy tissues or blood samples or cells obtained from tissue or blood and cultured outside the body, and thereby continue to possess as far as possible the original properties of the cells from the body. However, the cells may also be comparably treated according to the treatment of the patient outside the body.
**characterized in**
**that** for the efficacy measurement the individual drugs are measured in their most optimal concentration and their combination efficacy is combined by calculation,
wherein the statement of inefficacy or efficacy is determined with the aid of the theoretical distribution curve
and the optimum concentration of a single drug is **characterized in that** the PCIs (Percent Cell Growth Inhibition) or data of other efficacy readouts of differently tested living cells give a PCI range or data range of other efficacy readouts as wide as possible
and the theoretical distribution curve results from the mathematical combination of the distribution curves of all individual substances involved in the combination.
**characterized in**
**that** the arithmetical combination of the efficacies of the individual substances is determined by summing their PCIs (Percent Cell Growth Inhibition) or data of other efficacy readout methods and dividing by the number of substances of the combination,
wherein the theoretical distribution curve is **characterized by** calculating the mean values for the averages, medians and standard deviations of the distribution curves for the individual substances.

2. method according to claim 1
**characterized in**
**that** the efficacy is divided into at least two groups,
where this is, for example, efficacy and inefficacy, or
wherein one possibility is the subdivision into Extreme Resistance (ER), Medium Resistance (MR) and Weak Resistance (SR),
**characterized in that**
ER: PCIs are smaller mean (or median) - 1 × standard deviation,
MR: PCIs are greater than ER but less than mean (or median), and
SR: PCIs are greater than or equal to mean (or median)
or
the efficacy limits and groups can also be defined differently, but the limits and groups are then applied equally to all drugs or combinations tested.

3. method according to claim 1 to 2
**characterized in**
**that** the in vitro test is the CTR test or the identical EDR assay.

4. method according to claim 1 to 2 or 1 to 3
**characterized in**
**in that** the combination measurement is not carried out with individual substances, but by direct measurement of the combined substances,
wherein the combined substances are added to the assay in the same ratio and with reduced concentrations
where
in the case of 2 drug combinations, ½ of the concentrations of the individual substances are used in each case,
for 3 drug combinations, 1/3 of the concentrations of the individual substances are used in each case,
for 4 combinations ¼, for 5s 1/5 of the concentrations are used, and so on.

5. process according to claim 4
**characterized in**
**that** for the efficacy measurement the method according to claim 64 is used and the distribution curve for the efficacy is recorded with living cells, whereby the efficacy limits can thus be defined for this distribution curve.

6. method according to claim 4 to 5
**characterized in**
**that** the efficacy is subdivided into at least two groups,
one possibility being the subdivision into Extreme Resistance (ER), Medium Resistance (MR) and Weak Resistance (SR),
**characterized in that**
ER: PCIs are smaller mean (or median) - 1 × standard deviation,
MR: PCIs are greater than ER but less than mean (or median), and
SR: PCIs are greater than or equal to mean (or median)
or
the efficacy limits and groups can also be defined differently, but the limits and groups are then applied equally to all drugs or combinations tested.

7. method according to claim 1 to 6
for determining synergistic, additive and antagonistic effects of drug combinations. This is determined by comparing calculated efficacy (claims 1 to 3 and measured efficacy (claims 4 to 7).

8. method according to claim 1 to 7
**characterized in**
**that** the therapeutic drugs belong to the group of chemotherapeutic drugs (cytostatics) (such as carboplatin or paclitaxel), targeted drugs (such as tyrosine kinase inhibitors, humanized antibodies, monoclonal therapeutic antibodies or anti-hormone drugs such as tamoxifen) or immunotherapeutic drugs (such as checkpoint drugs such as pembrolizumab, nivolumab or atezolizumab) and are used for cancer treatment.

## Revendications

1. Procédé pour déterminer et sélectionner des combinaisons de médicaments thérapeutiques efficaces ou non efficaces pour des patients au moyen de tests individuels in vitro sur des échantillons de patients vivants avant ou pendant la thérapie,
**caractérisé en ce que**
que la mesure de l'efficacité est effectuée sur des cellules vivantes,
dans lesquelles les cellules vivantes sont constituées soit d'échantillons de tissus tumoraux frais, soit d'échantillons de tissus provenant d'autres maladies, soit de tissus sains, soit d'échantillons de sang, soit de cellules obtenues à partir de tissus ou de sang et cultivées à l'extérieur du corps, et continuent ainsi à posséder autant que possible les propriétés originales des cellules provenant du corps. Toutefois, les cellules peuvent également être traitées de manière comparable en fonction du traitement du patient à l'extérieur du corps.
**caractérisé en ce que**
que pour la mesure de l'efficacité, les médicaments individuels sont mesurés à leur concentration la plus optimale et que l'efficacité de leur combinaison est combinée par calcul,
où la déclaration d'inefficacité ou d'efficacité est déterminée à l'aide de la courbe de distribution théorique
et la concentration optimale d'un médicament individuel est **caractérisée par le fait que** les PCI (pourcentage d'inhibition de la croissance cellulaire) ou les données d'autres indicateurs d'efficacité de cellules vivantes testées différemment donnent une plage de PCI ou une plage de données d'autres indicateurs d'efficacité aussi large que possible
et la courbe de distribution théorique résulte de la combinaison mathématique des courbes de distribution de toutes les substances individuelles impliquées dans la combinaison.
**caractérisé en ce que**
que la combinaison arithmétique des efficacités des substances individuelles est déterminée en additionnant leurs PCI (pourcentage d'inhibition de la croissance cellulaire) ou les données d'autres méthodes de lecture de l'efficacité et en divisant par le nombre de substances de la combinaison,
dans lequel la courbe de distribution théorique est **caractérisée par** le calcul des valeurs moyennes des moyennes, des médianes et des écarts types des courbes de distribution pour les substances individuelles.

2. Méthode selon la revendication 1
**caractérisé en ce que**
que l'efficacité est divisée en au moins deux groupes,
où il s'agit, par exemple, de l'efficacité et de l'inefficacité, ou bien dans lequel une possibilité est la subdivision en résistance extrême (ER), résistance moyenne (MR) et résistance faible (SR),
**caractérisé en ce que**
ER : les ICP sont inférieures à la moyenne (ou à la médiane) - 1 × l'écart-type,
MR : les ICP sont supérieures à ER mais inférieures à la moyenne (ou à la médiane), et
SR : les ICP sont supérieures ou égales à la moyenne (ou à la médiane)
ou
les limites et les groupes d'efficacité peuvent également être définis différemment, mais les limites et les groupes sont alors appliqués de manière égale à tous les médicaments ou combinaisons testés.

3. Méthode selon la revendication 1 à 2
**caractérisé en ce que**
que le test in vitro est le test CTR ou le test EDR identique.

4. méthode selon la revendication 1 à 2 ou 1 à 3
**caractérisé en ce que**
**en ce que** la mesure de la combinaison n'est pas effectuée avec des substances individuelles, mais par mesure directe des substances combinées,
dans lequel les substances combinées sont ajoutées à l'essai dans le même rapport et avec des concentrations réduites
où
dans le cas de 2 combinaisons de médicaments, ½ des concentrations des substances individuelles sont utilisées dans chaque cas,
pour 3 combinaisons de médicaments, 1/3 des concentrations des substances individuelles sont utilisées dans chaque cas,
pour 4 combinaisons ¼, pour 5s 1/5 des concentrations sont utilisées, et ainsi de suite.

5. Procédé selon la revendication 4
**caractérisé en ce que**
que pour la mesure de l'efficacité, on utilise la méthode selon la revendication 64 et on enregistre la courbe de distribution de l'efficacité avec des cellules vivantes, les limites de l'efficacité pouvant ainsi être définies pour cette courbe de distribution.

6. procédé selon la revendication 4 à 5
**caractérisé en ce que**
que l'efficacité est subdivisée en au moins deux groupes,
une possibilité étant la subdivision en résistance extrême (ER), résistance moyenne (MR) et résistance faible (SR),
**caractérisé en ce que**
ER : les ICP sont inférieures à la moyenne (ou à la médiane) - 1 × l'écart-type, MR : les ICP sont supérieurs à ER mais inférieurs à la moyenne (ou à la médiane), et
SR : les ICP sont supérieures ou égales à la moyenne (ou à la médiane)
ou
les limites et les groupes d'efficacité peuvent également être définis différemment, mais les limites et les groupes sont alors appliqués de manière égale à tous les médicaments ou combinaisons testés.

7. Méthode selon les revendications 1 à 6
pour déterminer les effets synergiques, additifs et antagonistes des combinaisons de médicaments. Ceci est déterminé en comparant l'efficacité calculée (revendications 1 à 3 et l'efficacité mesurée (revendications 4 à 7).

8. Méthode selon la revendication 1 à 7
**caractérisé en ce que**
que les médicaments thérapeutiques appartiennent au groupe des médicaments chimiothérapeutiques (cytostatiques) (tels que le carboplatine ou le paclitaxel), des médicaments ciblés (tels que les inhibiteurs de tyrosine kinase, les anticorps humanisés, les anticorps thérapeutiques monoclonaux ou les médicaments anti-hormones tels que le tamoxifène) ou des médicaments immunothérapeutiques (tels que les médicaments de point de contrôle tels que le pembrolizumab, le nivolumab ou l'atezolizumab) et sont utilisés pour le traitement du cancer.
